# EUROPEAN PATENT APPLICATION

(11) **EP 4 623 814 A1**
(43) Date of publication of application: **01.10.2025**
(21) Application number: 24167143.7
(22) Date of filing: 28.03.2024
(51) Int. Cl.: A61B 5/0205, A61B 5/024, A61B 5/08, A61B 5/361, A61B 5/363, A61B 5/369, A61B 5/389, A61B 5/398, A61B 5/00

(54) **CONTINUOUS POSITIVE AIRWAY PRESSURE CPAP METHOD AND SYSTEM FOR ALERTING THE ONSET OF STROKE DURING SLEEP**

(71) Applicant: Koninklijke Philips N.V., 5656 AG Eindhoven (NL)
(72) Inventor: MENA BENITO, Maria Estrella, Eindhoven (NL); WESTERINK, Joanne Henriëtte Desirée Monique, Eindhoven (NL); WEIJSEN, Tim Elisabeth Joseph, 5656AG Eindhoven (NL); TSONEVA, Tsvetomira Kirova, Eindhoven (NL); KAHLERT, Joachim Johannes, Eindhoven (NL); JUAN, Elsa Joséphine Suzanne, Eindhoven (NL)
(74) Representative: Philips Intellectual Property & Standards

(57) **Abstract**

A method (84) for stroke onset alert for a continuous positive airway pressure (CPAP) system (12) comprises providing (86), via a blower (14) and a patient circuit (16), a pressurized flow of breathable gas to a patient interface device (18) configured for being worn by a subject (20). The method includes monitoring and recording (88), via a monitoring unit (28) integral with the patient interface device, a predetermined set of vital signs and physical symptoms of the subject relating to an onset of acute stroke attack or stroke episode during the delivery of the pressurized flow of breathable gas. In addition, the method comprises identifying (90), via a stroke onset detection module (34), an onset of stroke based on an analysis of the monitored and recorded set of vital signs and physical symptoms and outputting (92), via a stroke onset alert module (36), a stroke onset alert signal based on the identification of the onset of stroke via the stroke onset detection module.

## Description

### BACKGROUND

The present embodiments relate generally to positive airway pressure devices and more particularly, to a continuous positive airway pressure (CPAP) method and system for alerting the onset of stroke during sleep.

Sleep and cardiovascular risks relate to an onset of stroke as discussed briefly in the following. During sleep, physiological changes in respiratory and cardiovascular activity are predominantly sleep-cycle dependent and mediated by autonomic control. During non-rapid eye movement (NREM) sleep there is an increase in parasympathetic activity, while during rapid eye movement (REM) sleep there is a decrease in parasympathetic activity accounting for increase in cardiovascular activity. REM sleep is associated with greater tendency for upper airway collapse (due to decreased muscle tone), greater sympathetic activity and cardiovascular instability in healthy human subjects and more so in patients with obstructive sleep apnea (OSA). Any arousal during sleep results in an increase in respiratory and cardiovascular activity.

Accordingly, sleep apnea and its many associated awakenings cause pathological increases in sympathetic activity, contributing to autonomic dysregulation. This dysautonomia contributes to worsening of the cardiovascular risk profile. The worsening of cardiovascular profile is well known to increase the risk of stroke. During an OSA event, the increased negative intrathoracic pressure changes the systemic and pulmonary circulation, caused by a change in cardiac preload, cardiac afterload, stroke volume and blood pressure. By these OSA event triggered hemodynamic changes, a vulnerable plague/blood clot at a vessel wall can be dissolved, causing an ischemia in the heart, in the brain or in peripheral arteries.

Sleep apnea and stroke relate to each other as discussed briefly in the following. A link between sleep apnea, and sleep disordered breathing (SDB) in general, and cardiovascular risk factors and stroke have been described in several publications. Stroke is defined as an episode of neurologic dysfunction due to infarction or focal collection of blood within the central nervous system and represents the second cause of death and the third cause of disability worldwide. Crucially, it is estimated that between 8% and 30 % of all strokes occur during sleep. Early alerting is critical for stroke treatment; the key for effective stroke care is reducing the time from its onset to receiving medical treatment.

Sleep apnea is most likely to cause ischemic stroke (i.e., an interruption of blood supply to the brain), but it can also cause cardioembolic stroke. The more severe the sleep apnea, the greater the risk of stroke. The risk is higher in untreated or insufficiently treated OSA patients. Given the fact that obstructive sleep apnea (OSA) is a common co-morbid condition in stroke patients, it represents a very important risk factor for a cerebrovascular event (stroke). As a result, sleep apnea patients may live in constant fear of a possible stroke attack and worry about when a stroke could possibly happen, how to prevent it and how to prepare in case of attack. Stroke during sleep is especially challenging because of the time-sensitive nature of stroke treatment.

Nearby an ischemic stroke (independently of the affected region), the body releases neurotransmitters. These neurotransmitters cannot be measured directly but the sympathetic response is specific to stroke. Particularly during sleep, when muscles are relaxed, the response to the released neurotransmitter changes the sleep architecture, the breathing pattern and the hemodynamic parameters.

During sleep, stroke symptoms are not noticed by the patient, while up to 25% of stroke patients are reported to have been sleeping during the onset of a stroke. Early detection of stroke onset is critical in clinical practice to avoid unnecessary delays in treatment. Therefore, it would be desirable to have an approach to automatically detect and promptly alert the onset of a stroke attack during sleep.

Current standard technologies for stroke diagnosis are Computed Tomography (CT), Magnetic Resonance Imaging (MRI) and Ultrasound and blood measurements. These three technologies are good for stroke diagnosis in a hospital, but not appropriate for regular daily use at home or office for an early stroke-alarm and certainly not during sleep.

It is known that blood pressure (BP) spontaneously changes in most (acute ischemic) stroke patients. The BP pattern change is believed to be a natural compensatory auto-regulation mechanism to maintain cerebral blood flow and reduce neuronal death while the presence of the ultrahigh blood pressure for an extended period of time may inflict damage as well.

Other methods include electroencephalogram (EEG), brain wave, and impedance plethysmography (IPG) or photoplethysmography (PPG) on a patient's head. Wearing a device on the head during sleep (in addition to the CPAP device) is not appealing and not always possible for patients.

Accordingly, an improved method and apparatus for overcoming the problems in the art is desired.

### SUMMARY

According to one aspect, the embodiments of the present disclosure advantageously overcome the shortcomings and disadvantages of known methods and technologies described above. The embodiments of the present disclosure provide a system and method that alerts OSA patients of the onset of a stroke during sleep while receiving a pressurized flow of breathable gas via a CPAP device.

In one embodiment, a method for stroke onset alert for a continuous positive airway pressure (CPAP) system comprises providing, via a blower, a pressurized flow of breathable gas to a patient interface device, via a patient circuit fluidly coupled between the blower and the patient interface device. The patient interface device is configured for being worn by a subject during delivery of the pressurized flow of breathable gas. The method further comprises monitoring and recording, via a monitoring unit integral with the patient interface device, a predetermined set of vital signs and physical symptoms of the subject relating to an onset of acute stroke attack or stroke episode during the delivery of the pressurized flow of breathable gas. In addition, the method comprises identifying, via a stroke onset detection module, an onset of stroke based on an analysis of the monitored and recorded set of vital signs and physical symptoms and outputting, via a stroke onset alert module, a stroke onset alert signal based on the identification of the onset of stroke via the stroke onset detection module.

According to another embodiment, the method includes wherein the monitoring unit comprises a plurality of sensors for implementing one or more series of sensor modalities for determination of at least HRV (heart rate variability), HR (heart rate) and arrythmias (brain-heart interaction). In one embodiment, the plurality of sensors can be selected from the group consisting of photoplethsmography (PPG) sensors, EMG sensors, ECG sensors, and E-nose sensors. The PPG sensors may be integrated in different places of the patient interface device. The EMG sensors may be integrated in different places in the patient interface device. The different places can include a mask portion and a headgear strap portion. Furthermore, the EMG sensors can be adapted (i) to detect muscle tension in a predetermined number of places on the subject's face, (ii) to detect and distinguish muscle weakness and muscle atonia, and (iii) to enable a comparison of muscle tension on both sides of the subject's face based on a determination of individual differences and individual thresholds. The ECG sensors may be integrated in different places of the patient interface device, further wherein the ECG sensors can be adapted to determine heart rate variability HRV and HRV changes, and wherein an HRV decease is associated with acute stroke severity.

In another embodiment, the plurality of sensors includes at least one of (i) a breath pattern sensor modality adapted for determination of brain hypoxia based on a breathing pattern of the subject, or (ii) electronic nose, E-nose, sensors adapted for breath analysis and a determination of volatile markers and a given concentration linked to a moment of stroke onset. In yet another embodiment, the plurality of sensors includes at least one of (i) sensor modalities of EOG sensors and EEG sensors, wherein the EOG sensors are integrated in the patient interface device to detect a lateralized asymmetry of the subject's face, and wherein the EEG sensors are adapted for monitoring progressive changes in EEG morphology amplitude, and frequency that correlate with a severity of stroke, or (ii) a facial camera integrated in the patient interface device and adapted for use in detecting stroke onset based on a positioning of the subject's head and features of the subject's face, or (iii) simultaneous use of stimulating and recording electroneurography (ENoG) electrodes integrated in the patient interface device and adapted for use in detecting stroke onset. In a still further embodiment, the plurality of sensors includes ambulatory PSG sensors adapted for detecting sleep stages and apnea events.

According to yet another embodiment, the method includes wherein the stroke onset detection module comprises a dedicated algorithm to process and analyze the monitored and recorded set of vital signs and physical symptoms for identification of a presence (and time) of the onset of the acute stroke attack or stroke episode, and wherein the monitored and recorded set includes at least measured physical symptoms (e.g., facial symptoms) and hemodynamic and respiratory parameters. In another embodiment, the method includes wherein the stroke onset detection module is configured to use machine learning or predictive modeling algorithms for the analysis of the monitored and recorded set of vital signs and physical symptoms to identify the onset of stroke.

In a further embodiment, the method includes wherein the stroke onset alert signal comprises at least one of (i) a warning signal configured to carry out one or more of (a) alert the subject, (b) alert someone other than the subject, and (c) initiate an emergency call to a medical provider, emergency medical personnel, or rescue team, or (ii) one or more of a tactile, visual, and auditory alert for signaling an occurrence of the onset of stroke. According to a still further embodiment, the method includes wherein the stroke onset alert signal further comprises a follow-up information signal for use in seeking additional information, wherein the follow-up information signal is communicated to a remote audio-visual display device based on a given level of uncertainty in a prediction of the onset of stroke based on the identified onset of stroke.

According to one embodiment, a continuous positive airway pressure (CPAP) system with stroke onset alert comprises a blower adapted to provide a pressurized flow of breathable gas to a patient interface device via a patient circuit fluidly coupled between the blower and the patient interface device. The patient interface device is configured for being worn by a subject during delivery of the pressurized flow of breathable gas to an airway of the subject. The system further comprises a monitoring unit integral with the patient interface device adapted to monitor and record a predetermined set of vital signs and physical symptoms of the subject relating to an onset of acute stroke attack or stroke episode during the delivery of the pressurized flow of breathable gas. In addition, the system comprises a stroke onset detection module adapted to analyze the monitored and recorded set of vital signs and physical symptoms and a stroke onset alert module adapted to output a stroke onset alert signal based on the identification of the onset of stroke via the stroke onset detection module.

According to another embodiment, the monitoring unit comprises a plurality of sensors for implementing one or more series of sensor modalities for determination of at least HRV (heart rate variability), HR (heart rate) and arrythmias (brain-heart interaction). In another embodiment, the plurality of sensors are selected from the group consisting of photoplethsmography (PPG) sensors, EMG sensors, ECG sensors, and E-nose sensors. The PPG sensors may be integrated in different places of the patient interface device. The EMG sensors may be integrated in different places in the patient interface device. The different places may include a mask portion and a headgear strap portion. In addition, the EMG sensors can be adapted (i) to detect muscle tension in a predetermined number of places on the subject's face, (ii) to detect and distinguish muscle weakness and muscle atonia, and (iii) to enable a comparison of muscle tension on both sides of the subject's face based on a determination of individual differences and individual thresholds. The ECG sensors may be integrated in different places of the patient interface device, further wherein the ECG sensors are adapted to determine heart rate variability HRV and HRV changes, and wherein an HRV decease is associated with acute stroke severity.

In another embodiment, the plurality of sensors includes at least one of (i) a breath pattern sensor modality adapted for determination of brain hypoxia based on a breathing pattern of the subject, or (ii) electronic nose, E-nose, sensors adapted for breath analysis and a determination of volatile markers and a given concentration linked to a moment of stroke onset. In yet another embodiment, the plurality of sensors includes at least one of (i) sensor modalities of EOG sensors and EEG sensors, wherein the EOG sensors are integrated in the patient interface device to detect a lateralized asymmetry of the subject's face, and wherein the EEG sensors are adapted for monitoring progressive changes in EEG morphology amplitude, and frequency that correlate with a severity of stroke, or (ii) a facial camera integrated in the patient interface device and adapted for use in detecting stroke onset based on a positioning of the subject's head and features of the subject's face, or (iii) simultaneous use of stimulating and recording electroneurography (ENoG) electrodes integrated in the patient interface device and adapted for use in detecting stroke onset. In a still further embodiment, the plurality of sensors includes ambulatory PSG sensors adapted for detecting sleep stages and apnea events.

According to yet one embodiment, the CPAP system includes wherein the stroke onset detection module comprises a dedicated algorithm to process and analyze the monitored and recorded set of vital signs and physical symptoms for identification of a presence (and time) of the onset of the acute stroke attack or stroke episode, and wherein the monitored and recorded set includes at least measured physical symptoms (e.g., facial symptoms) and hemodynamic and respiratory parameters. In another embodiment, the CPAP system includes wherein the stroke onset detection module is configured to use machine learning or predictive modeling algorithms for the analysis of the monitored and recorded set of vital signs and physical symptoms to identify the onset of stroke.

In a further embodiment, the CPAP system includes wherein the stroke onset alert signal comprises at least one of (i) a warning signal configured to carry out one or more of (a) alert the subject, (b) alert someone other than the subject, and (c) initiate an emergency call to a medical provider, emergency medical personnel, or rescue team, or (ii) one or more of a tactile, visual, and auditory alert for signaling an occurrence of the onset of stroke. According to a still further embodiment, the CPAP system further includes wherein the stroke onset alert signal further comprises a follow-up information signal for use in seeking additional information, wherein the follow-up information signal is communicated to a remote audio-visual display device based on a given level of uncertainty in a prediction of the onset of stroke based on the identified onset of stroke.

The embodiments of the present disclosure advantageously solve or overcome the problems in the art by providing a method and system that measure physical symptoms and hemodynamic and respiratory parameters using a sensory unit integrated into a CPAP mask device (or patient interface device) and using stroke identification algorithms and machine learning to identify the onset of a stroke attack based upon the measured symptoms and parameters. The result of the detection is used to generate an alarm to alert the patient, a caregiver, or a medical professional as to the onset of a stroke attack during sleep, whereby the early detection of stroke onset during sleep advantageously avoids unnecessary delays in receiving prompt treatment.

Still further advantages and benefits will become apparent to those of ordinary skill in the art upon reading and understanding the following detailed description.

### BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing Figures, like reference numerals refer to like elements. In addition, it is to be noted that the Figures may not be drawn to scale.
Fig. 1 is a block diagram view of a continuous positive airway pressure (CPAP) device for alerting the onset of stoke during sleep according to an embodiment of the present disclosure;
Fig. 2 is a block diagram view of the controller of the CPAP device for alerting the onset of stoke during sleep of Fig. 1 in further detail according to an embodiment of the present disclosure;
Fig. 3 is a perspective view of a patient interface device of the CPAP device for alerting the onset of stoke during sleep, in the form of a total face mask, according to an embodiment of the present disclosure;
Fig. 4 is a perspective view of a patient interface device of the CPAP device for alerting the onset of stoke during sleep, in the form of a nasal mask, according to an embodiment of the present disclosure;
Fig. 5 is a perspective view of a patient interface device of the CPAP device for alerting the onset of stoke during sleep, in the form of a nasal mask with an attached patient circuit, according to another embodiment of the present disclosure;
Fig. 6 is a perspective view of a patient interface device of the CPAP device for alerting the onset of stoke during sleep, in the form of a full face mask, according to an embodiment of the present disclosure; and
Fig 7 is a flow diagram view of a method for alerting the onset of stoke during sleep with a continuous positive airway pressure CPAP device according to an embodiment of the present disclosure.

### DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

As discussed herein, obstructive sleep apnea (OSA) has been shown to increase the risk of stroke by its effect on vascular risk factors. Sleep apnea is most likely to cause ischemic stroke (*i.e.,* an interruption of blood supply to the brain), but it can also cause cardioembolic stroke. The more severe the sleep apnea, the greater the risk of stroke. Up to 25% of stroke patients are reported to have been sleeping during the onset of a stroke. During sleep, stroke symptoms often remain unnoticed by the patient. Detection of onset/time of stroke is critical to avoid unnecessary delays in treatment. The current approaches for detection of onset or presence of stroke are not suitable for a home setting, or are bulky. Therefore, it is highly desirable to have an approach to automatically detect and promptly alert the onset of a stroke attack during sleep.

The embodiments of the present disclosure advantageously provide an approach to alert CPAP users or their caregivers the onset of stroke or directly after (*i.e.,* within a predetermined amount of time from) the occurrence of the onset of stroke. The method and system according to the embodiment of the present disclosure measures physical symptoms and hemodynamic and respiratory parameters by means of a sensory unit integrated into the CPAP mask device and uses stroke identification algorithms and machine learning to identify the onset of stroke. The result of the detection is used to generate an alarm to alert the patient, caregiver, or medical professional.

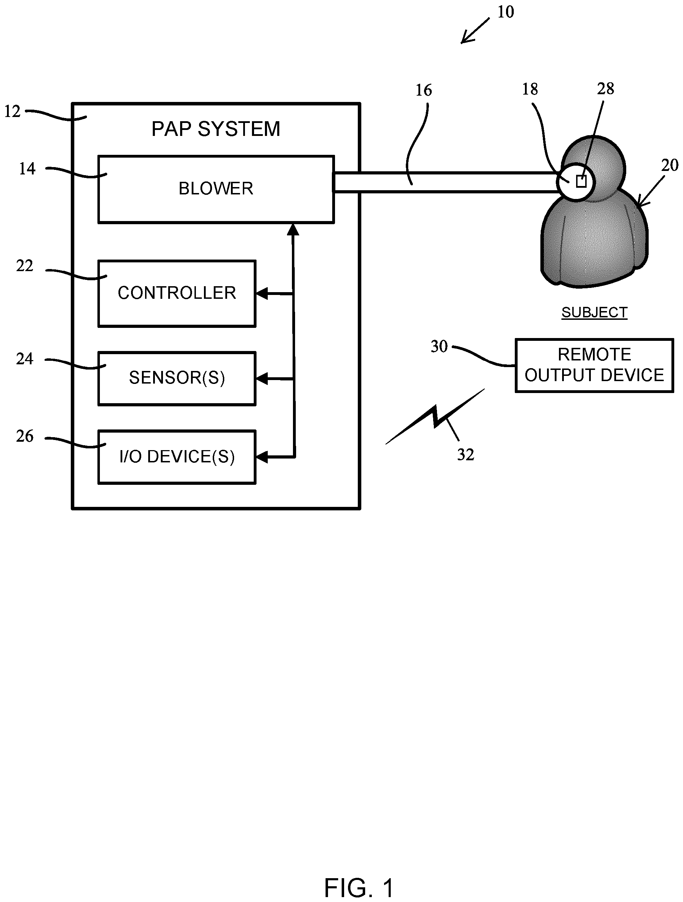

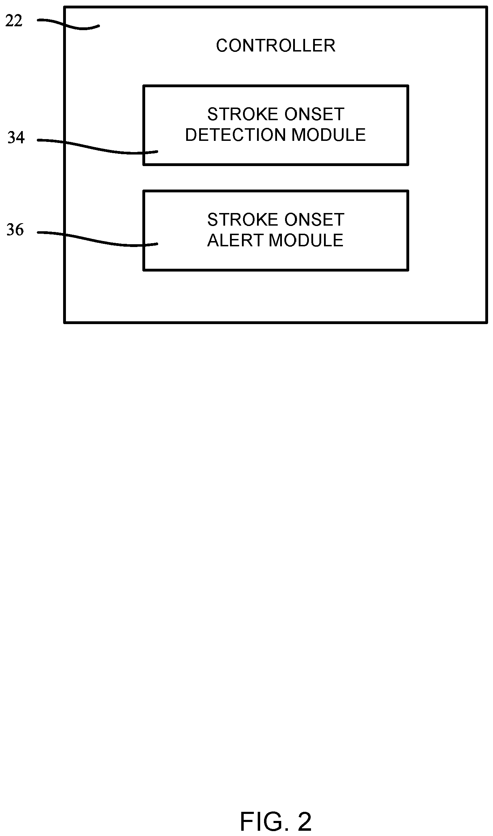

With reference now to Fig. 1, there is shown a block diagram view of a continuous positive airway pressure (CPAP) system or device 10 with stroke onset alert according to an embodiment of the present disclosure. The CPAP system 10 comprises a positive airway pressure device 12 having a gas flow generator or blower 14 configured to generate the flow of breathable gas. The gas flow generator 14 is configured to communicate the flow of breathable gas to a patient circuit 16 that includes a patient interface device or mask 18 to a patient or subject 20. The system further includes a controller 22 configured to implement one or more modules, as will be explained with reference to Fig. 2. In one embodiment, controller 22 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given PAP system implementation and/or application.

Controller 22 can further comprise one or more various modules configured to implement a given functionality as discussed herein. For example, the modules may comprise one or more of an integrated circuit, discrete analog or digital circuit components, hardware, software, firmware, or any combination thereof, for performing various functions as discussed herein, further according to the requirements of a given stroke onset alert implementation and/or application according to an embodiment of the present disclosure. In addition, one or more of the modules may further comprise various combinations of one or more of the various modules as discussed herein. Furthermore, it is understood that the described modules may be computer program modules which are rendered in a non-transitory computer-readable medium.

One or more sensor 24 (or sensors) are provided and configured to generate output signals related to at least one characteristic associated with at least one of respiratory, actigraphy, airflow, humidity, pressure, temperature, in-mask sensor signals, or any combination thereof. First sensor signals can comprise respiratory signals, actigraphy signals, or a combination of both. Second sensor signals can comprise airflow sensor signals, pressure sensor signals, in-mask sensor signals, IR sensor signals *(e.g.,* to detect a distance between a PAP mask and a user's forehead), or a combination thereof.

One or more input/output (I/O) device(s), collectively indicated by reference numeral 26, is representative of any number of suitable I/O devices for inputting or outputting one or more of an audible, visual, and/or tactile input or output, according to the requirements of a given PAP system implementation featured with stroke onset alert, as discussed herein. For example, input/output device(s) 26 can comprise one or more of an input/output device, a user interface, tactile output device, touch screen, optical display, microphone (*e.g.*, for receiving user voice commands/responses), keypad, keyboard, pointing device, image capture device, video camera, audio output device, and any combination thereof, according to the requirements of the given PAP system implementation featuring stroke onset alert. In another embodiment, input/output device 26 may include a mobile phone app configured for inputting or outputting one or more of an audible, visual, and/or tactile input or output, according to the requirements of a given PAP system implementation featuring stroke onset alert.

According to one embodiment, the CPAP system 10 further comprises a monitoring unit 28 that can include a plurality of sensors for implementing one or more series of sensor modalities for determination of at least HRV (heart rate variability), HR (heart rate) and arrythmias (brain-heart interaction). In another embodiment, the plurality of sensors are selected from the group consisting of photoplethsmography (PPG) sensors, EMG sensors, ECG sensors, and E-nose sensors. The PPG sensors may be integrated in different places of the patient interface device. The EMG sensors may be integrated in different places in the patient interface device. The different places may include a mask portion and a headgear strap portion. In addition, the EMG sensors can be adapted (i) to detect muscle tension in a predetermined number of places on the subject's face, (ii) to detect and distinguish muscle weakness and muscle atonia, and (iii) to enable a comparison of muscle tension on both sides of the subject's face based on a determination of individual differences and individual thresholds. The ECG sensors may be integrated in different places of the patient interface device, further wherein the ECG sensors are adapted to determine heart rate variability HRV and HRV changes, and wherein an HRV decease is associated with acute stroke severity.

In another embodiment, the monitoring unit 28 comprises a plurality of sensors that includes at least one of (i) a breath pattern sensor modality adapted for determination of brain hypoxia based on a breathing pattern of the subject, or (ii) electronic nose, E-nose, sensors adapted for breath analysis and a determination of volatile markers and a given concentration linked to a moment of stroke onset. In yet another embodiment, the plurality of sensors includes at least one of (i) sensor modalities of EOG sensors and EEG sensors, or (ii) a facial camera integrated in the patient interface device and adapted for use in detecting stroke onset based on a positioning of the subject's head and features of the subject's face, or (iii) simultaneous use of stimulating and recording electroneurography (ENoG) electrodes integrated in the patient interface device and adapted for use in detecting stroke onset.

The EOG sensors are integrated in the patient interface device to detect a lateralized asymmetry of the subject's face. The muscles of the upper side of the face is controlled bilaterally (*i.e.,* from both sides of the brain), whereas the muscles of the lower face are controlled ipsilaterally only (*i.e.,* from the opposite side of the brain). Lower face paralysis is more typical of ischemic stroke. The EEG sensors are adapted for monitoring progressive changes in EEG morphology amplitude, and frequency that correlate with a severity of stroke. In a still further embodiment, the plurality of sensors includes ambulatory PSG sensors adapted for detecting sleep stages and apnea events.

Furthermore, the monitoring unit 28 may include a facial camera integrated in the patient interface or mask: Special emphasis on "facial drop" on one side of the lower face particularly flattened nasolabial fold. The facial camera could take into account if the head is positioned slightly sidewards, which will influence the features of the face, especially is the person is older, and the skin is not so firm anymore. For high-risk cases, stroke onset could be detected using electroneurography (ENoG), where stimulating and recording electrodes are used simultaneously. ENoG electrodes can be integrated in the Philips Sleep head band or in the straps of a CPAP mask. The risk stratification can be used for an optimization of the CPAP therapy or to recommend a CPAP therapy in untreated patients.

The controller 22 and monitoring unit 28 may also include a machine learning unit, i.e., collection of information to determine trends and probability of stroke, potentially using data accumulated from other similar type patients. The computational unit 22 also can be used as predictive method/awareness method, such as the system provides information about the possibility of adapting - also to use as advice for - influencing parameters, such as bedtime or sleeping posture (since stroke probability might be found to be less for certain postures). Another potential way of influencing could be by preparing the user for sleep with certain methods (*e.g*., doing a paced breathing exercise before falling asleep). The CPAP device could offer and suggest such an intervention, and monitor across users whether such an intervention indeed lowers the chances of stroke, and if so, for which type of user. Other users with similar characteristics could then be advised to make use of this intervention as well. In accordance with one embodiment, the machine learning unit can comprise a machine learning model trained using the data collected by all sensors (*e.g.,* but not limited to, PPG, IPG, ECG, respiration, EEG, sleep architecture, EOG, camera, accelerometer, blood pressure sensor, environmental parameters, demographics, etc.). The algorithm can rely on supervised learning such as classification or regression-based methods (support vector machine/SVM, Linear discriminant analysis/LDA, logistic regression, etc.), unsupervised learning such as clustering and dimensionality reduction methods, or deep learning such as deep neural networks, recurrent neural networks, convolutional neural networks, GANs or similar. The machine learning unit can output probability of stroke or, given specific trends, advice on adapting some influencing parameters.

With reference still to Fig. 1, the continuous positive airway pressure (CPAP) device 10 with stroke onset alert can further comprise at least one remote output device 30. In Fig. 1, the lightning bolt identified via reference numeral 30 is indicative of a wired or wireless link (e.g., near field communications, Bluetooth^{™} or other wireless communications link) between a given component (*e.g.,* controller 22) of the PAP system 12 and the monitoring unit 28 and/or the at least one remote output device 30.

Whenever the system detects a stroke, the system can react in several ways: by alerting the bedpartner or housemate of the patient; by alerting medical staff; or by providing further info on a remote or bedside display, especially in cases where there is uncertainly of the prediction. Which options are preferred depends on the choice of patient and medical professional and can be indicated by means of an alert module in the device. The recorded information may be used for clinical decision support, for example, communication to clinician of moment and duration of stroke.

In another embodiment, the method and system can be implemented within the Philips SmartSleep or Deep sleep headband. Progressive changes in EEG morphology, amplitude, and frequency seem to correlate with the severity of stroke. Conditions of low oxygen such as ischemia, lead to abnormal changes in the EEG, gradually loosing high frequencies and increasing slower frequencies. This could also allow to flag potential stroke risk even before ischemic threshold leading to cell death is reached. This embodiment envisions the use of the existing sensors (EEG, accelerometer) in the SmartSleep band to monitor stroke onset and severity. However, additional sensors (*e.g.,* PPG, EMG or any of the examples given above) could improve stoke detection and allow for monitoring other cardiac conditions. The systems could send notifications to a paired mobile app and/or alert relatives/caregivers/medical professionals. The alerting notifications could be triaged based on the estimated stroke severity or the predicted risk of stroke.

Referring now to Fig. 2, there is shown a block diagram view of the controller 22 of the CPAP device 10 with stroke onset alert of Fig. 1 in further detail. In one embodiment, controller 22 comprises stroke onset detection module 34 adapted to analyze the monitored and recorded set of vital signs and physical symptoms. For example, stroke onset detection module 34 can include a dedicated algorithm to process and analyze the monitored and recorded set of vital signs and physical symptoms for identification of a presence (and time) of the onset of the acute stroke attack or stroke episode. The monitored and recorded set of vital signs and physical symptoms can include at least measured physical symptoms (*e.g.,* facial symptoms) and hemodynamic and respiratory parameters. In another embodiment, the stroke onset detection module 34 is configured to use machine learning or predictive modeling algorithms for the analysis of the monitored and recorded set of vital signs and physical symptoms to identify the onset of stroke.

With reference still to Fig. 2, controller 22 further comprises a stroke onset alert module 36 adapted to output a stroke onset alert signal based on the identification of the onset of stroke via the stroke onset detection module 34. In one embodiment, the stroke onset alert signal comprises at least one of (i) a warning signal configured to carry out one or more of (a) alert the subject, (b) alert someone other than the subject, and (c) initiate an emergency call to a medical provider, emergency medical personnel, or rescue team, or (ii) one or more of a tactile, visual, and auditory alert for signaling an occurrence of the onset of stroke. According to another embodiment, the stroke onset alert signal can further comprise a follow-up information signal for use in seeking additional information, wherein the follow-up information signal is communicated to a remote audio-visual display device based on a given level of uncertainty in a prediction of the onset of stroke based on the identified onset of stroke.

In other words, the method and system embodiments of the present disclosure measure physical symptoms and hemodynamic and respiratory parameters using a sensory unit integrated into the CPAP mask device or patient interface device and using stroke identification algorithms and machine learning to identify the onset of a stroke attack based upon the measured symptoms and parameters. The result of the detection is used to generate an alarm to alert the patient, a caregiver, or a medical professional.

In one embodiment, an input/monitoring unit (*e.g.,* monitoring unit 28) is located at the CPAP mask or patient interface device 18 and contains a series of sensors integrated to continuously monitor and record vitals signs and physical symptoms associated to the onset of an acute stroke attack. A computational unit (*e.g.,* controller 22) is configured to gather and analyze the monitored and recorded information from the input unit or input/monitoring unit. The dedicated algorithm is configured to process and analyze the monitored and recorded information from the input unit, *i.e.,* identification of the presence and time of onset of a stroke episode. The computational unit is in communication with the output unit/communication unit (*e.g.,* stroke onset alert module 36). The output unit is configured to provide the information to patient about the onset of stroke, a warning signal (*e.g.,* acoustic tone) alerts others, such as caregivers, bed partner, or directly put in an emergency call to a rescue team.

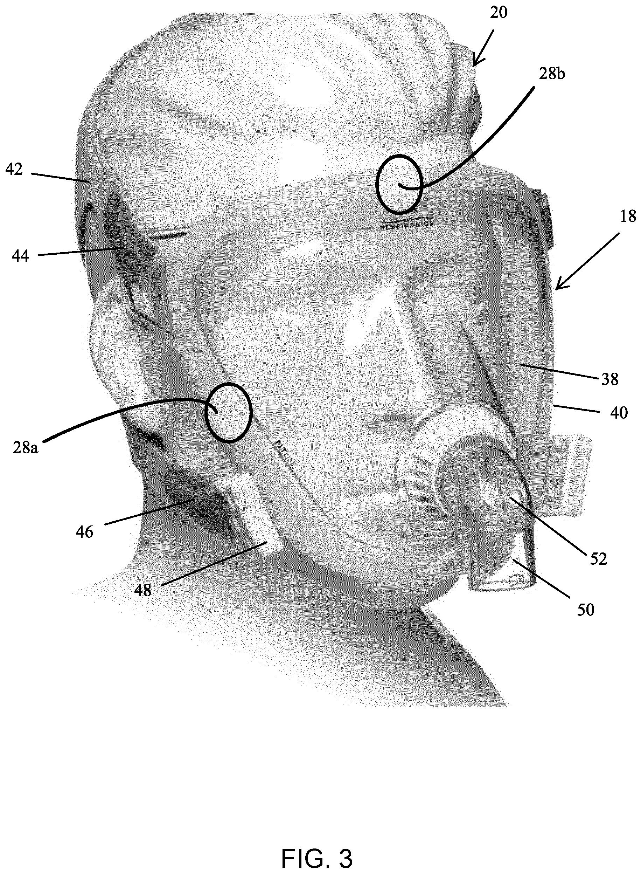

With reference now to Fig. 3, there is shown a perspective view of a patient interface device 18 of the CPAP device 10 with stroke onset alert, in the form of a total face mask 38, according to an embodiment of the present disclosure. As shown, the total face mask 38 doesn't contact the areas on and around the user's nose, but seals (*e.g.,* via an over-moulded cushion integrated into the mask frame 40 or faceplate) around the perimeter of the user's face. The total face mask 38 includes a large surface area to equalize pressure and flow inside the mask, minimizing eye irritation. The mask 38 further includes a headgear component 42 (*e.g.,* one or more low-stretch headgear straps that provide stability and a consistent fit) that physically couples to the total face mask via headgear adjustment tabs 44,46 located at upper and lower left and right sides of the face mask. The headgear tabs can include large inlaid headgear tabs for quick, easy adjustment and removal of the headgear. The upper adjustment tabs 44 attach to respective left and right locations on the frame 40 of the total face mask. The lower adjustment tabs 46 attach to respective lower left and right sides of the face mask frame via snap clips 48. The snap clips 48 enable quick set-up and removal (*e.g.,* subsequent to an initial fitting of the mask to the user's head) of the headgear strap 46 from the frame of the face mask 38 when a user desires to put on or take off the mask, respectively. The snap clips thus simplify set-up and eliminate the need to refit the mask after removal.

The total face mask 38 further includes a patient circuit elbow connector 50 equipped of an entrainment valve 52. The entrainment valve 52 allows access to room air if pressure drops below 3 cm H₂O. According to one embodiment, the monitoring device 28 includes a plurality of sensors 28a, 28b integrated in different places of the patient interface device 18, e.g., physically attached to the frame 40 of the total face mask 38.

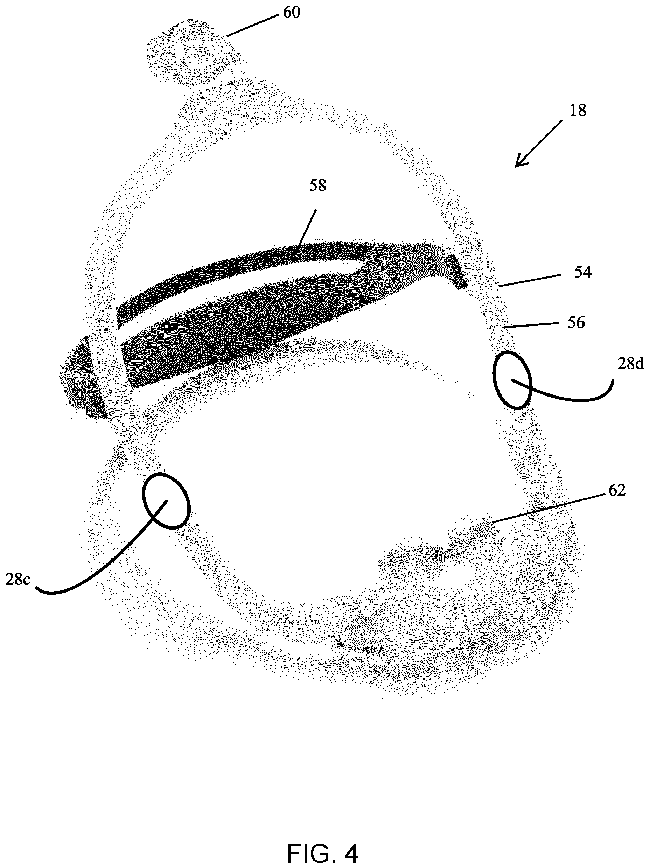

Turning now to Fig. 4, a perspective view is shown of a patient interface device 18 of the CPAP device 10 with stroke onset alert, in the form of a nasal mask 54, according to an embodiment of the present disclosure. The nasal mask 54 is configured to direct airflow through the frame 56 (*e.g.,* a soft silicone frame) so patients can wear glasses, watch TV and sleep comfortably. Mask 54 includes a headgear component 58 (*e.g.,* one or more low-stretch headgear straps and/or arms of a soft, slip-resistant design that provide stability and a consistent fit) that physically couples to the nasal mask frame 56 via headgear adjustment tabs located at left and right sides of the frame. A patient circuit elbow connector 60 (*i.e.,* tubing connection) is located at the top of the frame 56 (and thus on top of a user's head) which allows patients when wearing the mask to sleep in any position - stomach, side or back.

Still further, the nasal mask 54 comprises a silicone pillows cushion mask that conforms to various nose shapes and sizes. The nasal mask 54 is an under-the-nose design. It features a silicone pillows cushion 62 which is soft to the touch, softer than gel pillows, which deliver an exceptional nostril seal. According to one embodiment, the monitoring device 28 includes a plurality of sensors 28c, 28d integrated in different places of the patient interface device 18, e.g., physically attached to the frame 56 of the nasal mask 54.

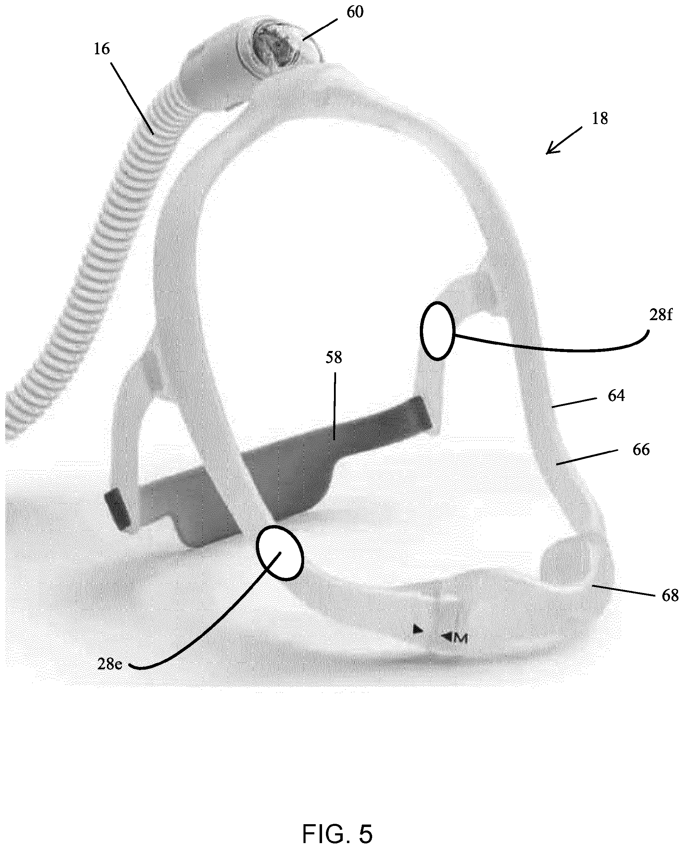

Turning now to Fig. 5, there is shown a perspective view of a patient interface device 18 of the CPAP device 10 with stroke onset alert, in the form of a nasal mask 64 with an attached patient circuit 16, according to another embodiment of the present disclosure. The embodiment of Fig. 5 is similar to that of Fig. 4, with the exception of an under-the-nose nasal cushion 68 in place of the nasal gel pillow cushion 62, according to an embodiment of the present disclosure. In this embodiment, the cushion opening is placed directly under the user's nostrils, whereby the cushion 62 hugs the user's nose and leaks will be minimal. According to one embodiment, the monitoring device 28 includes a plurality of sensors 28e, 28f integrated in different places of the patient interface device 18, e.g., physically attached to the frame 66 of the nasal mask 64.

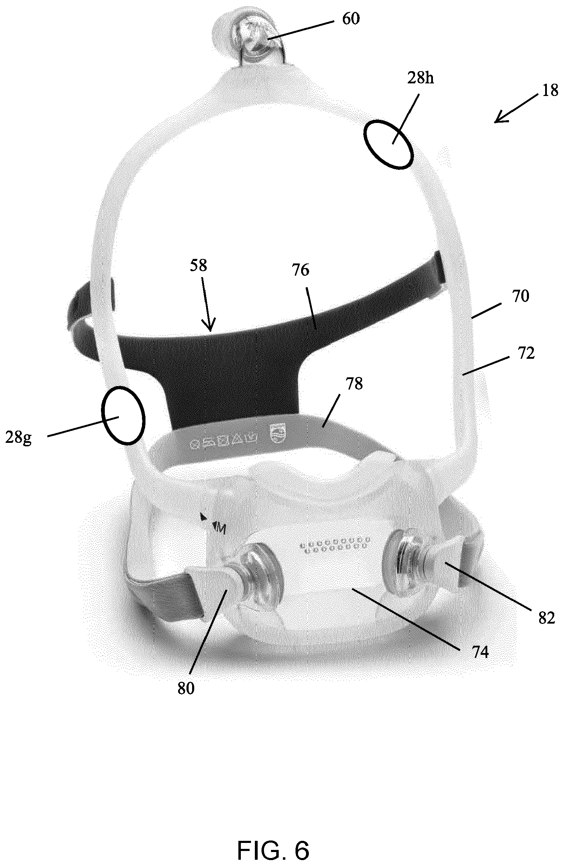

Turning now to Fig. 6, there is shown a perspective view of a patient interface device 18 of the CPAP device 10 with stroke onset alert, in the form of a full-face mask 70, according to an embodiment of the present disclosure. The embodiment of Fig. 6 is similar to that of Fig. 4, with the exception of a cushion 74 in place of the nasal gel pillow cushion 62, according to an embodiment of the present disclosure. In this embodiment, the cushion opening is directly under the user's nostrils, whereby the cushion 74 hugs the user's nose and mouth, and leaks will be minimal. In addition, the headgear component 58 includes a top strap 76 coupled to a bottom strap 78. The top strap 76 physically couples to the frame 72 of the full-face mask 70 via headgear adjustment tabs located at upper left and right sides of the mask. The lower strap 78 physically couples to the frame 72 of the full-face mask 70 via headgear clips 80,82 located at lower left and right sides of the mask.

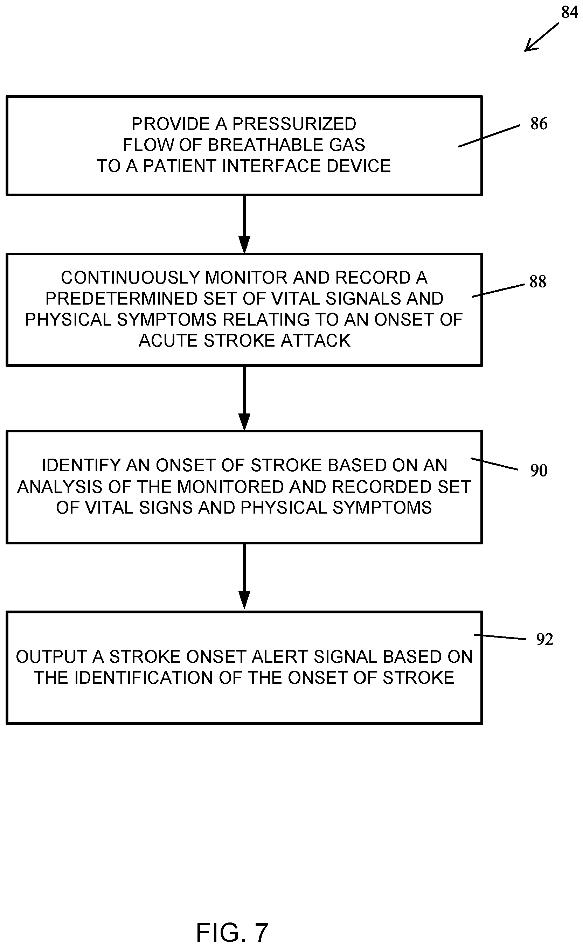

Fig. 7 is a flow diagram view of a method 84 for stroke onset alert for a continuous positive airway pressure (CPAP) system 10 according to an embodiment of the present disclosure. A first step 86 comprises providing, via a blower 14, a pressurized flow of breathable gas to a patient interface device 18, via a patient circuit 16 fluidly coupled between the blower and the patient interface device. The patient interface device 18 is configured for being worn by a subject 20 during delivery of the pressurized flow of breathable gas. The method further comprises a second step 88 of monitoring and recording, via a monitoring unit 28 integral with the patient interface device 18, a predetermined set of vital signs and physical symptoms of the subject relating to an onset of acute stroke attack or stroke episode during the delivery of the pressurized flow of breathable gas. In addition, the method comprises a third step 90 of identifying, via a stroke onset detection module 34, an onset of stroke based on an analysis of the monitored and recorded set of vital signs and physical symptoms and at step 92 outputting, via a stroke onset alert module 36, a stroke onset alert signal based on the identification of the onset of stroke via the stroke onset detection module.

Although only a few exemplary embodiments have been described in detail above, those skilled in the art will readily appreciate that many modifications are possible in the exemplary embodiments without materially departing from the novel teachings and advantages of the embodiments of the present disclosure. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A method (84) for stroke onset alert for a continuous positive airway pressure (CPAP) system (12), the method comprising:
providing (86), via a blower (14), a pressurized flow of breathable gas to a patient interface device (18) being worn by a subject during delivery of the pressurized flow of breathable gas, via a patient circuit (16) fluidly coupled between the blower and the patient interface device;
monitoring and recording (88), via a monitoring unit (28) integral with the patient interface device (18), a predetermined set of vital signs and physical symptoms of the subject relating to an onset of acute stroke attack or stroke episode;
identifying (90), via a stroke onset detection module (34), an onset of stroke based on an analysis of the monitored and recorded set of vital signs and physical symptoms; and
outputting (92), via a stroke onset alert module (36), a stroke onset alert signal based on the identification of the onset of stroke via the stroke onset detection module.

2. The method (84) of claim 1, wherein the monitoring unit (28) comprises a plurality of sensors (28a,28b,28c,28d,28e,28f,28g,28h) for implementing one or more series of sensor modalities for determination of at least heart rate variability, heart rate and arrythmias.

3. The method (84) of claim 1, wherein the plurality of sensors (28a,28b,28c,28d,28e, 28f,28g,28h) are selected from the group consisting of photoplethsmography PPG sensors, EMG sensors, ECG sensors, and E-nose sensors,
wherein the PPG sensors are integrated in different places of the patient interface device (18),
wherein the EMG sensors are integrated in different places in the patient interface device (18) that include a mask portion (40,56,66,72) and a headgear strap portion (42,58), further wherein the EMG sensors are adapted (i) to detect muscle tension in a predetermined number of places on the subject's face, (ii) to detect and distinguish muscle weakness and muscle atonia, and (iii) to enable a comparison of muscle tension on both sides of the subject's face based on a determination of individual differences and individual thresholds, and
wherein the ECG sensors are integrated in different places of the patient interface device (18), further wherein the ECG sensors are adapted to determine heart rate variability HRV and HRV changes, and wherein an HRV decease is associated with acute stroke severity.

4. The method (84) of claim 1, wherein the plurality of sensors (28a,28b,28c,28d,28e, 28f,28g,28h) includes at least one of
(i) a breath pattern sensor modality adapted for determination of brain hypoxia based on a breathing pattern of the subject, or
(ii) electronic nose, E-nose, sensors adapted for breath analysis and a determination of volatile markers and a given concentration linked to a moment of stroke onset.

5. The method (84) of claim 1, wherein the plurality of sensors (28a,28b,28c,28d,28e, 28f,28g,28h) includes at least one of
(i) sensor modalities of EOG sensors and EEG sensors, wherein the EOG sensors are integrated in the patient interface device to detect a lateralized asymmetry of the subject's face, and wherein the EEG sensors are adapted for monitoring progressive changes in EEG morphology amplitude, and frequency that correlate with a severity of stroke, or
(ii) a facial camera integrated in the patient interface device and adapted for use in detecting stroke onset based on a positioning of the subject's head and features of the subject's face, or
(iii) simultaneous use of stimulating and recording electroneurography ENoG electrodes integrated in the patient interface device and adapted for use in detecting stroke onset.

6. The method (84) of claim 1, wherein the plurality of sensors (28a,28b,28c,28d,28e, 28f,28g,28h) includes ambulatory PSG sensors adapted for detecting sleep stages and apnea events.

7. The method (84) of claim 1, wherein the stroke onset detection module (34) comprises a dedicated algorithm to process and analyze the monitored and recorded set of vital signs and physical symptoms for identification of a presence of the onset of the acute stroke attack or stroke episode, and wherein the monitored and recorded set includes at least measured physical symptoms and hemodynamic and respiratory parameters.

8. The method (84) of claim 1, wherein the stroke onset detection module (34) is configured to use machine learning or predictive modeling algorithms for the analysis of the monitored and recorded set of vital signs and physical symptoms to identify the onset of stroke.

9. The method (84) of claim 1, wherein the stroke onset alert signal comprises at least one of
(i) a warning signal configured to carry out one or more of (a) alert the subject, (b) alert someone other than the subject, and (c) initiate an emergency call to a medical provider, emergency medical personnel, or rescue team, or
(ii) one or more of a tactile, visual, and auditory alert for signaling an occurrence of the onset of stroke.

10. The method (84) of claim 1, wherein the stroke onset alert signal further comprises a follow-up information signal for use in seeking additional information, wherein the follow-up information signal is communicated to a remote audio-visual display device based on a given level of uncertainty in a prediction of the onset of stroke based on the identified onset of stroke.

11. A continuous positive airway pressure (CPAP) system (10) with stroke onset alert, the system comprising:
a blower (14) adapted to provide a pressurized flow of breathable gas to a patient interface device (18) for being worn by a subject during delivery of the pressurized flow of breathable gas, via a patient circuit (16) fluidly coupled between the blower and the patient interface device;
a monitoring unit (28) integral with the patient interface device (18) adapted to monitor and record a predetermined set of vital signs and physical symptoms of the subject relating to an onset of acute stroke attack or stroke episode;
a stroke onset detection module (34) adapted to analyze the monitored and recorded set of vital signs and physical symptoms; and
a stroke onset alert module (36) adapted to output a stroke onset alert signal based on the identification of the onset of stroke via the stroke onset detection module.

12. The system (10) of claim 11, wherein the monitoring unit (28) comprises a plurality of sensors (28a,28b,28c,28d,28e,28f,28g,28h) for implementing one or more series of sensor modalities for determination of at least heart rate variability, heart rate and arrythmias.

13. The system (10) of claim 11, wherein the plurality of sensors (28a,28b,28c,28d,28e, 28f,28g,28h) are selected from the group consisting of photoplethsmography PPG sensors, EMG sensors, ECG sensors, and E-nose sensors,
wherein the PPG sensors are integrated in different places of the patient interface device (18),
wherein the EMG sensors are integrated in different places in the patient interface device (18) that include a mask portion (40,56,66,72) and a headgear strap portion (42,58), further wherein the EMG sensors are adapted (i) to detect muscle tension in a predetermined number of places on the subject's face, (ii) to detect and distinguish muscle weakness and muscle atonia, and (iii) to enable a comparison of muscle tension on both sides of the subject's face based on a determination of individual differences and individual thresholds, and
wherein the ECG sensors are integrated in different places of the patient interface device (18), further wherein the ECG sensors are adapted to determine heart rate variability HRV and HRV changes, and wherein an HRV decease is associated with acute stroke severity.

14. The system (10) of claim 11, wherein the plurality of sensors (28a,28b,28c,28d,28e, 28f,28g,28h) includes at least one of
(i) a breath pattern sensor modality adapted for determination of brain hypoxia based on a breathing pattern of the subject, or
(ii) electronic nose, E-nose, sensors adapted for breath analysis and a determination of volatile markers and a given concentration linked to a moment of stroke onset.

15. The system (10) of claim 11, wherein the plurality of sensors (28a,28b,28c,28d,28e, 28f,28g,28h) includes at least one of
(i) sensor modalities of EOG sensors and EEG sensors, wherein the EOG sensors are integrated in the patient interface device (18) to detect a lateralized asymmetry of the subject's face, and wherein the EEG sensors are adapted for monitoring progressive changes in EEG morphology amplitude, and frequency that correlate with a severity of stroke, or
(ii) a facial camera integrated in the patient interface device and adapted for use in detecting stroke onset based on a positioning of the subject's head and features of the subject's face, or
(iii) simultaneous use of stimulating and recording electroneurography ENoG electrodes integrated in the patient interface device and adapted for use in detecting stroke onset.
